# EUROPEAN PATENT APPLICATION

(11) **EP 1 145 708 A2**
(43) Date of publication of application: **17.10.2001**
(21) Application number: 01107874.8
(22) Date of filing: 10.04.2001
(51) Int. Cl.: A61K 7/42, A61K 7/00

(54) **Cosmetic and use thereof**

(30) Priority: 10.04.2000 JP 2000107959
(71) Applicant: KAO CORPORATION, Chuo-ku, Tokyo (JP)
(72) Inventor: Hasebe, Yoshihiro, Wakayama-shi, Wakayama (JP); Nambu, Hiromi, Wakayama-shi, Wakayama (JP); Takahashi, Toshie, Sumida-ku, Tokyo (JP); Osumi, Shinzo, Sumida-ku, Tokyo (JP)
(74) Representative: HOFFMANN - EITLE

(57) **Abstract**

A cosmetic comprising: component (A): water-insoluble polymer particles having an average particle size of from 1 to 40 µm, comprising a shell part including a hydrophilic polymer and a core part including a hydrophobic polymer, and carrying an ultraviolet ray preventing agent on their surface or inside; and component (B): a cationic surface active agent. This cosmetic may be used as an ultraviolet ray preventing agent.

## Description

### FIELD OF THE INVENTION

This invention relates to a cosmetic having excellent used feeling, in which its ultraviolet ray preventing agent efficiently adheres to hair or the skin.

### BACKGROUND OF THE INVENTION

It is known that, when exposed excessively to ultraviolet rays of sunlight, they induce inflammatory symptoms such as red spots and water blisters on the skin and become a cause of generating spots, wrinkles, freckles and further skin cancers and they also induce discoloration of hair and accelerate fading of dyed hair, so that a variety of ultraviolet ray preventing agents have been developed and blended in cosmetics for the purpose of protecting the skin and hair from ultraviolet rays.

However, when an ultraviolet ray preventing agent is applied to the skin, it is washed out together with sweat and water and almost all of it is washed out in the case of a dosage form which is used by rinsing out, such as hair cosmetics, so that there is a problem that its effect cannot sufficiently be obtained.

In that case, there are possible methods such as blending of a large amount of an ultraviolet ray preventing agent in cosmetics and fixation of the ultraviolet ray preventing agent with other adhesive components, but sufficient effect has not been obtained from any of them.

On the other hand, it is important as cosmetics that not only they have water resistance but also they are not sticky to the touch and have excellent used feeling such as moist feeling or dry feeling.

### SUMMARY OF THE INVENTION

A primary object of the invention is to provide a cosmetic having high ultraviolet ray preventing effect, in which its ultraviolet ray preventing agent can be adhered to hair or the skin efficiently without spoiling the touch.

The present inventors have found that a cosmetic having high ultraviolet ray preventing effect, in which its ultraviolet ray preventing agent can be adhered to hair or the skin efficiently without spoiling the touch due to improved dispersion stability of the composition, can be obtained by blending particles of an ultraviolet ray preventing agent-carried water-insoluble polymer having a specified shell part and core part with a specified surface active agent.

Accordingly, the invention provides a cosmetic which comprises the following components (A) and (B);
(A) water-insoluble polymer particles having an average particle size of from 1 to 40 µm, having a shell part which uses a hydrophilic polymer as a constituting component and a core part that uses a hydrophobic polymer as a constituting component, and carrying an ultraviolet ray preventing agent on their surface or inside, and
(B) a cationic surface active agent.

### DETAILED DESCRIPTION OF THE INVENTION

The hydrophilic monomer which constitutes the hydrophilic polymer of shell part in the water-insoluble polymer particles of component (A) preferably has a solubility of 0.1 g or more at 20°C based on 100 g of water, and its examples include methyl acrylate, hydroxyethyl acrylate, polyethylene glycol monoacrylate, glycidyl acrylate, methyl methacrylate, hydroxypropyl methacrylate, polyethylene glycol monomethacrylate, methacrylamide, N,N-dimethylacrylic amide, vinyl acetate and crotonic acid; unsaturated carboxylic acid monomers such as acrylic acid, methacrylic acid, itaconic acid and maleic acid; unsaturated sulfonic acid monomers such as styrenesulfonic acid, 2-acrylamido-2-methylpropanesulfonic acid and vinylsulfonic acid; and organic acids having reactive vinyl group including unsaturated phosphoric acid monomers such as vinyl phosphate and bis(methacryloxyethyl) phosphate or salts thereof (alkali metal salts such as of sodium and potassium and ammonium salts can be cited as the salts) . These hydrophilic monomers can be used alone or as a mixture of two or more. In addition, chitosan for example can also be cited as the hydrophilic polymer.

Among them, it is desirable to use chitosan and a polymer of an organic acid having a reactive vinyl group or a salt thereof as the constituting component.

The chitosan to be used herein is a deacetylated product of chitin having a (1→4)-2-acetamido-2-deoxy-β-D-glucan structure, and a chitosan derivative having a (1→4)-2-amino-2-deoxy-β-D-glucan structure, in which a portion of deacetylated amino groups or a portion of hydroxyl groups inside the same molecule are chemically modified by acylation reaction, etherification reaction, esterification reaction or other reaction, is also included. In general, a portion of acetamido groups in the naturally existing chitin are present as un-acetylated amino groups, but the chitosan to be used in the invention has a de-acetylation degree of 30% or more.

Also, the organic acid having reactive vinyl group or a salt thereof is a water-soluble organic acid having one or more reactive vinyl groups and one or more acidic groups in the molecule, which can dissolve chitosan to make it into an aqueous solution, and among its examples, an unsaturated carboxylic acid monomer having a relatively low acidity is desirable, and methacrylic acid having particularly low acidity of polymer is most desirable.

Together with these organic acid having reactive vinyl group or salts thereof, various acids can be optionally mixed, and their examples include inorganic acids such as hydrochloric acid, sulfuric acid and phosphoric acid and organic acids such as formic acid, acetic acid, citric acid, succinic acid, oxalic acid, glycolic acid, dichloric acid and trifluoroacetic acid.

The hydrophobic monomer which constitutes the hydrophobic polymer of core part in the water-insoluble polymer particles of component (A) preferably has a solubility of less than 0.1 g at 20°C based on 100 g of water, and its examples include styrene and divinylbenzene; acrylic acid esters or methacrylic acid esters represented by a general formula (1) 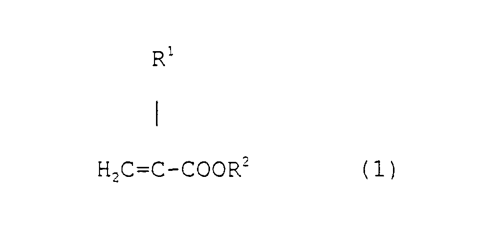 (wherein R¹ represents hydrogen atom or methyl group, and R² represents a straight or branched chain hydrocarbon radical having from 4 to 30 carbon atoms); a fluorine based monomer such as trifluoroethyl methacrylate; and a silicone macromonomer containing, e.g., a vinyl group. These hydrophobic monomers can be used alone or as a combination of two or more.

The surface or inside of the water-insoluble polymer particles composed of a shell part and core part carries an ultraviolet ray preventing agent.

As the ultraviolet ray preventing agent, ultraviolet ray absorbing agents and ultraviolet ray scattering agents can generally be exemplified, and any one of them can be used in the cosmetic of the invention.

The ultraviolet ray absorbing agents are divided into oily and water-soluble products, and examples of the oily ultraviolet ray absorbing agents include benzoic acid based agents such as p-aminobenzoic acid (to be referred to as PABA hereinafter), glyceryl PABA, ethyldihydroxypropyl PABA, N-ethoxylate PABA ethyl ester and N-dimethyl PABA ethyl ester; anthranilic acid base agents such as homomenthyl-N-acetyl anthranilate; salicylic acid base agents such as amyl salicylate, menthyl salicylate, homomenthyl salicylate and octyl salicylate; cinnamic acid base agents such as octyl cinnamate, ethyl-4-isopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethylhexyl-p-methoxy cinnamate, 2-ethoxyethyl-p-methoxy cinnamate and cyclohexyl-p-methoxy cinnamate; and benzophenone base agents such as 2,4-dihydroxybenzophenone, 2,2'-dihydroxy-4-methoxybenzophenone, 2,2'-dihydroxy-4,4'-dihydroxybenzophenone, 2-hydroxy-4-methoxybenzophenone, 2-hydroxy-4-methoxy-4'-methylbenzophenone, 2-hydroxy-4-methoxybenzophenone and 4-phenylbenzophenone; as well as 3-(4'-methylbenzylidene)-dl-camphor, 3-benzylidene-dl-camphor, urocanic acid ethyl ester, 2-phenyl-5-methylbenzoxazole, 2,2'-hydroxy-5-methylphenylbenzotriazole, 2-(2'-hydroxy-5-t-octylphenyl)benzotriazole, dibenzalazine, dianisoylmethane, 4-methoxy-4'-t-butyldibenzoylmethane, benzenebis-1,3-diketone derivatives described in JP-A-2-212579 (the term "JP-A" as used herein means an "unexamined published Japanese patent application") and benzoylpinacolone derivatives described in JP-A-3-220153, and examples of the water-soluble ultraviolet ray absorbing agents include diethanolamine p-methoxycinnamate, sodium 2-hydroxy-4-methoxybenzophenone-5-sulfonate, tetrahydroxybenzophenone, methylherperidine, sodium 3-hydroxy-4-methoxycinnamate, sodium ferulate, urocanic acid, and animal and plant extracts having ultraviolet ray absorption action, such as of yarrow, aloe, marshmallow, burdock and sage.

Examples of the ultraviolet ray scattering agents include titanium oxide, fine particle titanium oxide (JP-A-57-67681), zinc oxide, fine zinc flower (JP-A-62-228006), thin section zinc oxide (JP-A-1-175921), iron oxide, fine particle iron oxide, cerium oxide and zirconium oxide, which may be surface-treated with a compound such as silicone, metal soap, N-acylglutamic acid or perfluoroalkyl phosphate. Their form, size and shape are not particularly limited, and they may be used in a shape such as sol.

Among these ultraviolet ray preventing agents, 2-ethylhexyl-p-methoxycinnamate (trade name Parsol MCX) and 4-methoxy-4'-t-butyldibenzoylmethane (trade name Parsol 1789) are particularly desirable.

Such ultraviolet ray preventing agents can be used in combination of two or more and their combination and amounts to be contained are decided depending on the desired ultraviolet ray preventing effect, but when the ultraviolet ray preventing effect is taken into consideration, it is desirable to contain from 5 to 50% by weight, particularly from 20 to 35% by weight, of them in polymer particles.

The water-insoluble polymer particles of component (A) having a shell part and a core part and containing an ultraviolet ray preventing agent is produced, for example, by using a hydrophobic monomer, a hydrophilic monomer and an ultraviolet ray preventing agent, forming a core part by polymerizing the hydrophobic monomer in the presence of an oil soluble initiator and forming a shell part by polymerizing the hydrophilic monomer in the presence of a water-soluble initiator. Illustratively, they can be produced for example by a method in which monomer droplets having an average particle size of 10 µm or less, obtained by emulsifying and dispersing a hydrophilic monomer, a hydrophobic monomer, an ultraviolet ray preventing agent, an oil soluble initiator and if necessary a non-polymerizable hydrophobic substance in water, are polymerized and then core part and shell part of the polymer particles are formed by adding a water-soluble initiator and carrying out the polymerization, or by a method in which polymer particles having an average particle size of 10 µm or less and having compatibility with a hydrophobic monomer are swelled by adding the hydrophobic monomer, an ultraviolet ray preventing agent and if necessary a non-polymerizable hydrophobic substance and then added to a hydrophilic monomer aqueous solution, and the thus obtained swelled polymer particles are subjected to the polymerization of core part and shell part in the presence of an oil soluble initiator and if necessary a water-soluble initiator.

Among these particles, when the shell part uses chitosan and a polymer of an organic acid having a reactive vinyl group or a salt thereof as the constituting component, they can be produced for example by the following production methods (1) and (2).

### [Production method (1)]

A method in which monomer droplets having an average particle size of 10 µm or less, obtained by emulsifying and dispersing chitosan (a) , an organic acid having reactive vinyl group or a salt thereof (b), a hydrophobic monomer (c), an ultraviolet ray preventing agent (d), an oil soluble initiator (e) and if necessary a non-polymerizable hydrophobic substance (f) in water, are polymerized and then core part and shell part of the polymer particles are formed by adding a water-soluble initiator (g) and carrying out the polymerization.

In this production method (1), blending ratio of the chitosan (a) and the organic acid having reactive vinyl group or a salt thereof (b) is not particularly limited when an acid having no double bond is jointly used, and when an acid having no double bond is not jointly used, it is desirable to use from 0.75 to 10 mol of the organic acid having reactive vinyl group or a salt thereof (b), based on 1 mol of the monosaccharide unit of chitosan. When the amount is less than 0.75 mol, chitosan will show a tendency of not completely dissolving in water.

Regarding blending ratio of the chitosan (a) and the hydrophobic monomer (c), it is desirable to use the chitosan (a) in an amount of from 1 to 5, 000 parts by weight, particularly from 2 to 300 parts by weight, based on 100 parts by weight of the hydrophobic monomer (c) . When amount of the chitosan (a) is less than 1 part by weight based on 100 parts by weight of the hydrophobic monomer (c), it will cause a tendency in that the performance of chitosan is not sufficiently exerted, and when the amount exceeds 5, 000 parts by weight, it will cause a tendency of spoiling stability of the polymer emulsion.

In this production method (1), it is desirable to use the oil soluble initiator (e) within the range of from 0.05 to 10.0 parts by weight based on 100 parts by weight of the hydrophobic monomer (c).

The oil soluble initiator (e) is a compound which initiates addition polymerization of monomers by radical decomposition with heat or in the presence of a reductive substance, and a compound such as an oil soluble peroxide or azobis compound is generally used. Its examples include organic peroxides such as lauroyl peroxide and benzoyl peroxide and azo compounds such as 2,2'-azobisisobutyronitrile, 2,2'-azobis(2,4-dimethylvaleronitrile) and 2,2'-azobis(2-methylbutyronitrile). These initiators can be used as a mixture of two or more.

The polymerization in this production method (1) can be carried out in the presence or absence of a surface active agent. Examples of the surface active agent to be used include general anionic, cationic, nonionic and amphoteric surface active agents.

Examples of the anionic surface active agent include dodecyl sulfate, dodecylbenzene sulfonate and sulfate salt of polyoxyethylene nonylphenyl ether.

Examples of the nonionic surface active agent include polyoxyethylene alkyl ether, polyoxyethylene alkylphenyl ether and polyoxyethylene-polyoxypropylene block copolymer.

Examples of the cationic surface active agent include octadecyltrimethylammonium chloride.

Examples of the amphoteric surface active agent include alkyldimethylaminoacetic acid betaine and 2-alkyl-N-carboxy-N-hydroxy imidazolinium betaine.

However, since amphoteric ions are present in the polymer particles, a nonionic surface active agent is desirable when stability of the particles is taken into consideration. Amount of the surface active agent to be used is not particularly limited, but it is desirable use it within the range of from 0.1 to 20 parts by weight based on 100 parts by weight of the hydrophobic monomer (c).

Also in this production method (1), a water-soluble polymer can be used as an emulsifying agent. Examples of the water-soluble polymer include polyvinyl alcohol and its derivatives, starch and its derivatives and cellulose derivatives.

These surface active agents and water-soluble polymers can be used as a mixture of two or more.

Also in this production method (1), a non-polymerizable hydrophobic substance (f) can be used when the hydrophobic monomer (c) is emulsified, by mixing the substance with the hydrophobic monomer (c).

In this case, plasticizers and chain transfer agents can be exemplified as the non-polymerizable hydrophobic substance (f). Examples of the plasticizer include esters such as phthalic acid diester, adipic acid diester and succinic acid diester; benzoic acid esters such as sucrose benzoate; phosphoric acid esters such as tricresyl phosphate; and diethylbenzene.

In this case, amount of the non-polymerizable hydrophobic substance (f) to be used is preferably from 0 to 90% by weight, particularly from 1 to 50% by weight, based on the hydrophobic monomer (c) which forms a core.

In this production method (1), an O/W emulsion containing monomer droplets having an average particle size of 10 µm or less is prepared by mixing the chitosan (a), the organic acid having reactive vinyl group or a salt thereof (b), the hydrophobic monomer (c), the ultraviolet ray preventing agent (d), the oil soluble initiator (e) and if necessary the non-polymerizable hydrophobic substance (f) with water, and emulsifying the mixture by mechanical agitation using an emulsifier. The average particle size of monomer droplets exceeding 10 µm is not desirable, because average particle size of polymer particles of the thus obtained polymer emulsion exceeds 30 µm.

Examples of the emulsifier to be used include an ultrasonic homogenizer, a homo-mixer, a milder, an atomizer, an (ultra)high-pressure homogenizer, a nanomizer system and a membrane emulsifying apparatus. In this connection, it is desirable to select the solid contents concentration at the time of emulsification within the range of from 1 to 60% by weight.

In this production method (1), the polymerization is carried out by heating the O/W emulsion prepared in the manner. Though the polymerization temperature varies depending on the kind of initiator, a range of approximately from 40°C to 90°C is suitable. Also, though the polymerization time varies depending on the monomer, initiator species and reaction temperature, it is generally from 1 to 24 hours. In addition, in order to polymerize the organic acid having reactive vinyl group or a salt thereof (b) of the shell part in the monomer droplets, the water-soluble initiator (g) may be added during polymerization of the core part or after completion of polymerization of the core part.

The water-soluble initiator (g) to be used in this case is a compound which initiates addition polymerization of monomers by radical decomposition with heat or in the presence of a reductive substance, and a compound such as a water-soluble peroxodisulfuric acid salt, peroxide or azobis compound is generally used. Its examples include peroxodisulfuric acid salts such as potassium persulfate and ammonium persulfate, peroxides such as hydrogen peroxide and t-butyl hydroperoxide and azo compounds such as 2,2'-azobis-2-amidinopropane salts (V-50) and 4,4'-azobis-4-cyanopentanoic acid. As occasion demands, it can be used as a redox based initiator in combination with a reducing agent.

Amount of the water-soluble initiator (g) to be used is preferably within the range of from 0.05 to 20% by weight based on the organic acid or a salt thereof (b). In addition, in order to increase the chitosan content in the polymer particles, an aqueous solution of the chitosan (a) and the organic acid having reactive vinyl group or a salt thereof (b) may be further added during the polymerization.

### [Production method (2)]

A method in which polymer particles (h) having an average particle size of 10 µm or less and having compatibility with the hydrophobic monomer (c) are swelled by adding the hydrophobic monomer (c), the ultraviolet ray preventing agent (d) and if necessary the non-polymerizable hydrophobic substance (f) and then added to an aqueous solution of the chitosan (a) and the organic acid having reactive vinyl group or a salt thereof (b), and the thus obtained swelled polymer particles are subjected to the polymerization of core part and shell part in the presence of the oil soluble initiator (e) and if necessary the water-soluble initiator (g).

This production method (2) is a method in which the polymer particles (h) having an average particle size of 10 µm or less are swelled by the ultraviolet ray preventing agent (d) and the hydrophobic monomer (c) , or the polymer particles (h) having an average particle size of 10 µm or less are swelled by the non-polymerizable hydrophobic substance (f), the ultraviolet ray preventing agent (d) and the hydrophobic monomer (c) , and then the polymerization is carried out in the presence of an initiator using the chitosan (a) and the organic acid having reactive vinyl group or a salt thereof (b) as the protective colloid. In this case, the average particle size of the polymer particles (h) exceeding 10 µm is not desirable, because average particle size of polymer particles of the thus obtained polymer emulsion exceeds 30 µm.

The polymer particles (h) to be used are not particularly limited, with the proviso that they are polymer particles having the average particle size of 10 µm or less, preferably from 0.01 to 10 µm, and a material such as anionic polymer emulsion, cationic polymer emulsion, nonionic polymer emulsion, polymer fine particles or micro gel can be used. Though the composition of these polymer particles is not particularly limited, those which have high compatibility with the hydrophobic monomer (c) are desirable.

In this production method (2), it is desirable to use the hydrophobic monomer (c) in an amount of from 5 to 20,000% by weight, particularly from 10 to 1,000% by weight, as the solid content based on the polymer particles (h) . When amount of the hydrophobic monomer is less than 5% by weight, it will cause a tendency in that binding of the core part and shell part becomes insufficient, and the amount exceeding 20,000% by weight will result in insufficient stability.

Examples of the method for swelling the polymer particles (h) by the ultraviolet ray preventing agent (d) and the hydrophobic monomer (c), or as occasion demands, by the non-polymerizable hydrophobic substance (f), the ultraviolet ray preventing agent (d) and the hydrophobic monomer (c), include a method in which the hydrophobic monomer (c) and the ultraviolet ray preventing agent (d) are added to the polymer particles (h) and a method in which the hydrophobic monomer (c) and the ultraviolet ray preventing agent (d), or the hydrophobic monomer (c), the ultraviolet ray preventing agent (d) and a water-soluble organic solvent, are emulsified using a surface active agent and added to the polymer particles (h) dispersed in water. In this case, the non-polymerizable hydrophobic substance (f) may be added together with the hydrophobic monomer (c), and amount of the non-polymerizable hydrophobic substance (f) is preferably from 0 to 90% by weight, particularly from 1 to 50% by weight, based on the hydrophobic monomer (c).

When the polymer particles (h) swelled by the ultraviolet ray preventing agent (d) and the hydrophobic monomer (c) or by the non-polymerizable hydrophobic substance (f), the ultraviolet ray preventing agent (d) and the hydrophobic monomer (c) are dispersed in water, the surface active agent exemplified in the production method (1) may be used. The surface active agent to be used is any one of anionic, cationic and nonionic surface active agents. Its amount to be used is desirably from 1 to 50% by weight based on the polymer particles.

In this production method (2), after swelling the polymer particles (h) in the above manner by the ultraviolet ray preventing agent (d) and the hydrophobic monomer (c) or by the non-polymerizable hydrophobic substance (f), the ultraviolet ray preventing agent (d) and the hydrophobic monomer (c), an aqueous solution of the chitosan (a) and the organic acid having reactive vinyl group or a salt thereof (b) is added thereto, and the thus obtained swelled polymer particles are subjected to the polymerization of core part and shell part in the presence of the oil soluble initiator (e) and if necessary the water-soluble initiator (g).

In this production method (2), blending amounts of the chitosan (a) and the organic acid having reactive vinyl group or a salt thereof (b), the ultraviolet ray preventing agent (d) and the initiator are similar to those of the production method (1).

The polymer emulsion obtained by a method such as the above production method (1) or (2) can be neutralized using a base. Examples of the base include ammonia, methylamine, ethylamine, propylamine, butylamine, isobutylamine, hexylamine, octylamine, amino-modified silicone, ethylenediamine, propylenediamine, butylenediamine, sodium hydroxide, potassium hydroxide, calcium hydroxide, sodium carbonate, sodium alkoxide and potassium alkoxide.

Amount of the base to be added is preferably from 0.1 to 2.0 mol %, particularly from 0.6 to 1.2 mol %, based on the organic acid polymer in the emulsion.

From the viewpoints of adhesion and extension properties and the touch, it is desirable that the thus obtained water-insoluble polymer particles in the polymer emulsion have an average particle size of from 1 to 40 µm, preferably from 5 to 30 µm, particularly from 10 to 20µm. In this connection, the average particle size of polymer particles was measured by a laser diffraction type particle size distribution measuring apparatus (LA-910, mfd. by HORIBA).

Two or more types of the water-insoluble polymer particles of the component (A) can be blended, and it is desirable that they are contained in an amount of from 0.05 to 30% by weight, preferably from 0.1 to 20% by weight, more preferably from 1 to 10% by weight. Also, the thus obtained polymer emulsion of water-insoluble polymer particles can be used as such.

The cosmetic of the invention further contains a cationic surface active agent as the component (B). By blending the component (B), the dispersion stability can be improved and the used feeling such as moist feeling and dry feeling can be added.

The following can be exemplified as the cationic surface active agent, and, according to the invention, these cationic surface active agents may be used alone or as a mixture of two or more and it is desirable that at least one higher alcohol is further blended in combination with such a cationic surface active agent.

As the cationic surface active agent, a quaternary ammonium salt represented by a general formula (2-A) and an acid-neutralized amide amine compound represented by a general formula (2-B) can be exemplified. 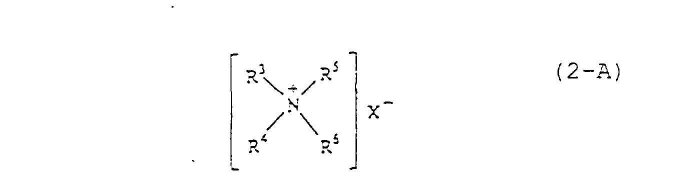 [In this formula, 1 to 3 of R³, R⁴, R⁵ and R⁶ indicate an alkyl or alkenyl group having from 8 to 22 carbon atoms, which may contain an amido group, an ester group or an ether group, and the rest indicate a group selected from hydrogen atom, an alkyl group having from 1 to 4 carbon atoms, a hydroxyalkyl group having from 1 to 4 carbon atoms, a benzyl group and polyoxyalkylene(C₁₋₄) group, and X indicates a halogen atom, an alkyl sulfate residue or an alkyl phosphate residue.]

In the above general formula (2-A), an alkyl group having from 8 to 18 carbon atoms is desirable as the alkyl or alkenyl group having from 8 to 22 carbon atoms. Also in the general formula (2-A), a compound in which one or two of R³ to R⁶ is an alkyl or alkenyl group having from 8 to 18 carbon atoms is desirable from the viewpoints of solubility and stability, and a compound in which these groups are an alkyl or alkenyl group having from 14 to 22 carbon atoms is desirable from the viewpoint of the touch.

R⁷CONH(CH₂)nN(R⁸)₂ (2-B)

[In this formula, R⁷ is the residue of a higher fatty acid having from 11 to 21 carbon atoms, R⁸ is an alkyl or hydroxyalkyl group having from 1 to 4 carbon atoms, and n is an integer of from 2 to 4.]

Examples of the acid to be used for the neutralization include inorganic acids such as hydrochloric acid and phosphoric acid, organic acids such as citric acid, lactic acid and malic acid and amino acids such as glutamic acid and α-alanine.

Illustrative examples of the cationic surface active agent include cetyltrimethylammonium bromide, behenyltrimethylammonium chloride, distearyldimethylammonium chloride, tricetylmethylammonium chloride, oxyethylalkylammonium phosphate, cocoalkylmethylbis(polyethoxyethanol) ammonium chloride, palmitoylethylhydroxyethylammoniummethyl sulfate, stearyldimethylbenzylammonium chloride, N-methyl-N,N-bis (long chain alkanoyloxyethyl)-N-(2-hydroxyethyl) ammoniummethyl sulfate, N-dimethyl-N,N-bis (long chain fatty acid amidepropyl) ammoniummethyl sulfate, N-dimethyl-N-(stearoyloxyethyl)-N-(stearic acid amidopropyl)ammoniummethyl chloride, diethylaminoethylamide stearate hydrochloride, dimethylaminopropylamide stearate hydrochloride, diethylaminopropylamide stearate glutamate, dimethylaminopropylamide palmitate lactate and dimethylaminopropylamide behenate malate, which may be used alone or as a mixture.

Regarding the higher alcohol, a higher alcohol having a straight or branched chain alkyl or alkenyl group of 12 to 26 carbon atoms is desirable, and its illustrative examples include cetyl alcohol, stearyl alcohol, arachidic alcohol, behenyl alcohol, caranavil alcohol and seryl alcohol. A higher alcohol having a straight chain alkyl group of 14 to 22 carbon atoms is more desirable.

Regarding the component (B) content in the cosmetic of the invention, it is desirable to include it in an amount of from 0.05 to 30 by weight, particularly from 0.1 to 10% by weight from the viewpoints of dispersion stability and the touch, in the total composition.

In addition to the above components, as occasion demands, the cosmetic of the invention can be blended optionally with surface active agents such as anionic surface active agent, nonionic surface active agent and amphoteric surface active agent; hydrocarbons such as liquid paraffin and vaseline; lanolin derivatives such as liquid lanolin and lanolin fatty acid; phospholipid such as lecithin; sterol such as cholesterol and derivatives thereof; collagen digested peptide derivatives; perfluoropolyether; oils and fats such as higher alcohol higher fatty acid esters and higher fatty acids; animal and plant oils and fats such as mink oil and olive oil; drug effective agents such as anti-dandruff agent, germicide and vitamins; polyols such as glycerol and propylene glycol; antiseptics such as parabens; thickeners such as water-soluble polymer; and coloring agents such as dye and pigment, ultraviolet ray preventing agents (ultraviolet ray absorbing agent and ultraviolet ray scattering agent), plant extracts, astringents, plasticizers, perfumes and coloring matter.

The cosmetic of the invention can be produced in accordance with usual methods, for example, as hair cosmetics such as hair rinse, hair treatment, hair conditioner, hair pack, hair lotion and hair shampoo; and skin cosmetics such as lotion, foundation, skin milk and skin cream, particularly desirably as a dosage such as hair rinse, hair treatment, hair conditioner, hair pack or hair shampoo, which is used by applying to hair and then rinsing it out.

### EXAMPLES

Production Example 1 (Production of ultraviolet ray preventing agent including particles A)

A 45 g portion of water was added to 2.5 g of commercially available chitosan (SK-10, mfd. by Koyo Chemical; de-acetylation degree 85 to 88%, weight average molecular weight 130,000), and 1.0 g of methacrylic acid (equimolar to the monosaccharide unit of chitosan) was added thereto and dissolved by stirring at 60°C, thereby preparing a chitosan/methacrylic acid aqueous solution.

Next, 13 g of stearyl methacrylate and 0.8 g of lauroyl peroxide were put into a 1 liter capacity glass beaker and dissolved, the solution was added to the chitosan/methacrylic acid aqueous solution to which 150 g of ion exchange water had been added, and then the mixture was treated for 5 minutes with an ultrasonic homogenizer (mfd. by Nihon Seiki Seisakusho) while stirring, thus obtaining an emulsion having an average particle size of 10 µm. This was mixed with a dispersion of 5 g of an ultraviolet ray preventing agent (2-ethylhexyl-p-methoxy cinnamate; trade name Parsol MCX), 2 g of Emulgen-220 and 20 g of ion exchange water and then mixed with monomer droplets by treating for 1 minute with the ultrasonic homogenizer while stirring. This emulsion was transferred into another reaction vessel equipped with a stirrer, a condenser and a nitrogen introducing tube, and, after replacing the atmosphere in the tube with nitrogen, heated to an inner temperature of 75°C while stirring. After 2 hours of polymerization with stirring, a solution prepared by dissolving 0.1 g of ammonium persulfate in 10 g of water was added thereto and 2 hours of additional reaction was carried out to obtain a single distribution polymer emulsion having an average particle size of 10 µm, in which the ultraviolet ray preventing agent was included.

When the thus obtained emulsion was freeze-dried and the resulting particles were observed by SEM or TEM, they were spherical and the outer shell layer and inner layer were confirmed, so that a core-shell type structure was confirmed. Also, it was confirmed that the ultraviolet ray preventing agent was enclosed in the core-shell particles.

The thus obtained particles were used in Inventive Example 1 and Comparative Example 3.

### Production Example 2 (Production of ultraviolet ray preventing agent including particles B)

An emulsion was prepared in the same manner as described in Production Example 1, except that methacrylic acid was changed to 0.5 g, a chitosan aqueous solution was prepared by adding 0.8 g of acrylic acid, and stearyl methacrylate was changed to 7.6 g of 2-ethylhexyl acrylate.

Also, the ultraviolet ray preventing agent of Production Example 1 was changed to 5 g of 4-methoxy-t-butyldibenzoylmethane (trade name Parsol 1789), 8 g of Emulgen-220 and 80 g of ion exchange water and charged, thereby obtaining a single distribution polymer emulsion having an average particle size of 10 µm, in which the ultraviolet ray preventing agent was included.

When structure of the thus obtained particles was analyzed in the same manner as described in Production Example 1, a core-shell type structure was confirmed and it was confirmed that the ultraviolet ray preventing agent was enclosed in the core-shell particles.

The thus obtained particles were used in Inventive Example 2.

### Production Example 3

The ultraviolet ray preventing agent of Production Example 1 was changed to 5 g of titanium oxide (MT-500B, mfd. by TEIKA), 8 g of Emulgen-220 and 80 g of ion exchange water and charged, thereby obtaining a single distribution polymer emulsion having an average particle size of 10 µm, which carried the ultraviolet ray preventing agent.

When structure of the thus obtained particles was analyzed in the same manner as described in Production Example 1, a core-shell type structure was confirmed and it was confirmed that the ultraviolet ray preventing agent was carried on the surface of the core-shell particles.

The thus obtained particles were used in Inventive Example 3.

### Production Example 4

A single distribution polymer emulsion having an average particle size of 3 µm, carrying the ultraviolet ray preventing agent, was obtained by repeating the same procedure of Production Example 1, except that the ultrasonic homogenizer treatment was changed to 10 minutes and an emulsion having an average particle size of 3 µm was obtained.

The thus obtained particles were used in Inventive Examples 4, 5 and 6.

### Inventive Examples 1 to 3 and Comparative Examples 1 to 3

Hair rinses having compositions shown in Table 1 were produced in the usual way, and their dispersion stability, ultraviolet ray preventing effect and used feeling were evaluated by the following evaluation methods. The results are also shown in Table 1.

### Inventive Examples 4 to 6

Hair rinses having compositions shown in Table 2 were produced in the usual way, and the same evaluation of Inventive Examples 1 to 3 was carried out. The results are also shown in Table 2.

### (Evaluation Methods)

### (1) Dispersion stability:

Each cosmetic after formulation was allowed to stand at room temperature for 1 day, and its appearance was evaluated with the naked eye by the following criteria.
A; Uniform with no problems in appearance.
B; A preparation causing aggregation and separation shows irregular appearance.

### (2) Ultraviolet ray preventing effect:

A 1 g portion of a bundle of hair was shampooed, treated with 0.2 g of each hair rinse, uniformly smoothed with fingers and then immediately rinsed thoroughly with hot water and dried. This was exposed to ultraviolet rays for a predetermined period of time using a solar simulator YSS-150 manufactured by Yamashita Denso, and then color difference (ΔE) from the color of original hair was obtained by measuring with a colorimeter CR-300 manufactured by Minolta Camera to carry out the evaluation by the following criteria. Smaller ΔE value indicates higher ultraviolet ray preventing effect.
A; ΔE is less that 3.
B; ΔE is 3 or more and less than 5.
C; ΔE is 5 or more.

### (3) Used feeling:

A 10 g portion of a bundle of hair was shampooed, treated with 1 g of each hair rinse, uniformly smoothed with fingers and then immediately rinsed thoroughly with hot water and dried. The touch of these samples was evaluated by 5 monitors and judged by the following criteria.
A; Four or more of the five monitors felt moist feeling or smooth feeling.
B; Two or three of the five monitors felt moist feeling or smooth feeling.
C; Zero or one of the five monitors felt moist feeling or smooth feeling.

**Table 1**

| | Inventive | | | Comparative | | |
|---|---|---|---|---|---|---|
| Component (% by weight) | 1 | 2 | 3 | 1 | 2 | 3 |
| Stearyltrimethylammonium chloride | 1 | 1 | 1 | 1 | 1 | - |
| Ultraviolet ray preventing agent including polymer particles (Inventive Example 1) | 5 | - | - | - | - | 5 |
| Ultraviolet ray preventing agent including polymer particles (Inventive Example 2) | - | 5 | - | - | - | - |
| Ultraviolet ray preventing agent including polymer particles (Inventive Example 3) | - | - | 2.5 | - | - | - |
| Parsol MCX | - | - | - | 0.5 | - | - |
| Parsol 1789 Polyoxyethylene (4) polyoxypropylene (30) | - | - | - | - | 0.5 | - |
| stearyl ether | 1 | 1 | 1 | 1 | 1 | 1 |
| Isostearyl glyceryl ether | 0.5 | 0.5 | 0.5 | 0.5 | 0.5 | 0.5 |
| Hydroxyethyl cellulose | 0.5 | 0.5 | 0.5 | 0.5 | 0.5 | 0.5 |
| Isopropyl myristate | 2 | 2 | 2 | 2 | 2 | 2 |
| Cetanol | 3 | 3 | 3 | 3 | 3 | - |
| Propylene glycol | 2 | 2 | 2 | 2 | 2 | 2 |
| Glycerol | 3 | 3 | 3 | 3 | 3 | 3 |
| Perfume | ap* | ap | ap | ap | ap | ap |
| Ion exchange water | b * | b | b | b | b | b |
| Dispersion stability | A | A | A | A | A | C |
| Ultraviolet ray preventing effect | A | A | A | C | C | A |
| Used feeling | A | A | A | A | A | C |
| ap: appropriate amount | | | | | | |
| b: balance | | | | | | |

**Table 2**

| Component (% by weight) | Inventive Example | | |
|---|---|---|---|
| | 4 | 5 | 6 |
| Behenyltrimethylammonium chloride | 1.04 | 1.6 | |
| N-Methyl-N,N-bis (oleyloxyethyl)-N-(2-hydroxyethyl) ammoniummethyl sulfate | | 0.8 | |
| Dimethylaminopropylamide stearate hydrochloride | | - | 1 |
| Dialkyl (C12-15) dimethylammonium chloride | 0.1 | 0.1 | |
| Ultraviolet ray preventing agent including polymer particles (Production Example 4) | 3 | 3 | 5 |
| Hydroxyethyl cellulose | 0.4 | 0.4 | 0.5 |
| Isopropyl palmitate | 0.5 | 0.5 | 2 |
| Cetanol | 3.6 | 7.2 | 3 |
| Propylene glycol | 1 | 1 | 1 |
| Polyethylene glycol | 0.1 | 0.1 | 0.1 |
| Benzyloxyethanol | 0.3 | 0.3 | 0.3 |
| Dimethicone | 0.5 | 1 | 1 |
| Perfume | ap* | ap | ap |
| Ion Exchange water | balance | balance | balance |
| Dispersion stability | A | A | A |
| Ultraviolet ray preventing effect | A | A | A |
| Used feeling | A | A | A |
| ap: appropriate amount | | | |

As is evident from the results shown in Tables 1 and 2, all of the cosmetics of the invention showed excellent dispersion stability and ultraviolet ray preventing effect, and they also showed good used feeling without damaging hair.

The cosmetics of the invention have excellent dispersion stability and can adhere ultraviolet ray preventing agent to hair or the skin efficiently without spoiling the touch. Thus, they can provide high ultraviolet ray preventing effect even by a single use and show excellent used feeling without damaging hair.

While the invention has been described in detail and with reference to specific examples thereof, it will be apparent to one skilled in the art that various changes and modifications can be made therein without departing from the spirit and scope thereof.

The entire disclosure of each and every foreign patent application from which the benefit of foreign priority has been claimed in the present application is incorporated herein by reference, as if fully set forth.

## Claims

1. A cosmetic comprising:
component (A) : water-insoluble polymer particles having an average particle size of from 1 to 40 µm, comprising a shell part including a hydrophilic polymer and a core part including a hydrophobic polymer, and carrying an ultraviolet ray preventing agent on their surface or inside; and
component (B): a cationic surface active agent.

2. The cosmetic according to claim 1, wherein the shell part of component (A) comprises chitosan and a polymer of an organic acid having a reactive vinyl group or a salt thereof.

3. The cosmetic according to claim 1 or 2, wherein the shell part of component (A) comprises a polymer of chitosan and methacrylic acid.

4. The cosmetic according to any one of the claims 1 to 3, wherein component (B) contains at least one of:
a quaternary ammonium salt represented by formula (2-A): 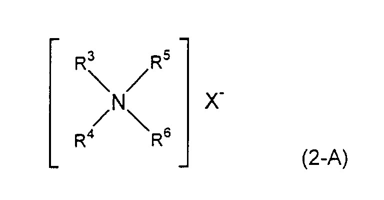 wherein one to three of R³, R⁴, R⁵ and R⁶ each independently represent an alkyl or alkenyl group having from 8 to 22 carbon atoms, which may contain at least one of an amido group, an ester group and an ether group, and the rest each independently represent a group selected from a hydrogen atom, an alkyl group having from 1 to 4 carbon atoms, a hydroxyalkyl group having from 1 to 4 carbon atoms, a benzyl group and polyoxyalkylene(C₁₋₄) group; and X represents a halogen atom, an alkyl sulfate residue or an alkyl phosphate residue; and
an acid-neutralized product of an amide amine compound represented by formula (2-B):
R⁷CONH(CH₂)ₙN(R⁸)₂ (2-B)
wherein R⁷ represents the residue of a higher fatty acid having from 11 to 21 carbon atoms, R⁸ represents an alkyl or hydroxyalkyl group having from 1 to 4 carbon atoms, and n is an integer of from 2 to 4.

5. The cosmetic according to any one of the claims 1 to 4,wherein component (B) further comprises at least one higher alcohol.

6. Use of the cosmetic as defined in any one of the claims 1 to 5 as an ultraviolet ray preventing agent
